# EUROPEAN PATENT SPECIFICATION

(11) **EP 1 835 922 B1**
(45) Date of publication and mention of the grant of the patent: **20.05.2009**
(21) Application number: 05823965.8
(22) Date of filing: 20.12.2005
(51) Int. Cl.: A61K 31/7076, A61K 39/395, A61P 37/02, C07K 16/28

(54) **COMBINATION TREATMENT FOR MULTIPLE SCLEROSIS**
KOMBINATIONSTHERAPIE GEGEN MULTIPLE SKLEROSE
TRAITEMENT COMBINÉ DE LA SCLÉROSE EN PLAQUES

(30) Priority: 22.12.2004 US 638674 P; 22.12.2004 EP 04106910
(43) Date of publication of application: 26.09.2007
(73) Proprietor: Merck Serono SA, 1267 Coinsins, Vaud (CH)
(72) Inventor: DE LUCA, Giampiero, CH-1231 Conches (CH)
(74) Representative: Sutcliffe, Nicholas Robert
(86) International application number: PCT/EP2005/056943
(87) International publication number: WO 2006/067134

(56) References cited:
- WO-A2-03/072040
- US-A- 5 506 214
- LEARY S M ET AL: "CURRENT MANAGEMENT OF MULTIPLE SCLEROSIS" INTERNATIONAL JOURNAL OF CLINICAL PRACTICE, MEDICON INTERNATIONAL, ESHER, GB, vol. 54, no. 3, April 2000 (2000-04), pages 161-169, XP008018314 ISSN: 1368-5031
- TUBRIDY N ET AL: "THE EFFECT OF ANTI-ALPHA4 INTEGRIN ANTIBODY ON BRAIN LESION ACTIVITY IN MS" NEUROLOGY, LIPPINCOTT WILLIAMS & WILKINS, PHILADELPHIA, US, vol. 53, no. 3, 11 August 1999 (1999-08-11), pages 466-472, XP008050557 ISSN: 0028-3878
- BEUTLER E ET AL: "The treatment of chronic progressive multiple sclerosis with cladribine." PROCEEDINGS OF THE NATIONAL ACADEMY OF SCIENCES OF THE UNITED STATES OF AMERICA. 20 FEB 1996, vol. 93, no. 4, 20 February 1996 (1996-02-20), pages 1716-1720, XP002339785 ISSN: 0027-8424 cited in the application

## Description

### BACKGROUND OF THE INVENTION

### 1. Field of the Invention

This invention relates to a method of treating multiple sclerosis (MS) by administering to a patient in need thereof a combination of agents characterized by a first agent that has the pharmacological activity of reducing the number of monocytes and a second agent that blocks the adhesion of monocytes and leukocytes to endothelial cells.

### 2. Description of Related Art

Multiple sclerosis (MS) is a progressive neurological autoimmune disease that affects 250,000 to 350,000 people in the United States alone. It is the result of demyelination in the brain and spinal cord. It is the most widely known chronic inflammatory demyelinating disease of the central nervous system (CNS) in humans. The onset of the disease typically occurs between the ages of twenty to forty, with twice as many women affected as men. MS onset is defined by the occurrence of the first neurological symptoms of CNS dysfunction. Symptoms resulting from this demyelination include weakness, visual impairment, incoordination, parathesis (abnormal tingling), CNS inflammation, brain atrophy and cognitive impairment. Sexual impairment and sphincter dysfunction may also be seen. The course of disease is unpredictable, but often progresses through a cycle of exacerbation of symptoms followed by remission. Remissions vary in length and may last several years but are infrequently permanent. Advances in cerebrospinal fluid (CSF) analysis and magnetic resonance imaging (MRI) have simplifed the diagnostic process and facilitated early diagnosis (Noseworthy et al., NEJM, 2000, 343(13):938-952).

MS can be categorized by four courses of disease: relapsing-remitting (RR)-, secondary progressive (SP)-, primary progressive (PP)- and progressive relapsing (PR)- MS. More than 80% of patients will initially display a RR course with the clinical exacerbation of neurological symptoms, followed by a recovery that may or may not be complete (Lublin and Reingold, Neurology, 1996, 46:907-911). During RRMS, accumulation of disability results from incomplete recovery from relapses. Approximately half of the patients with RRMS switch to SPMS within about ten years after disease onset. During the SP phase, worsening of the disability results from the accumulation of residual symptoms after exacerbation but also from insidious progression between exacerbations (*Lublin and Reingold, supra*). Ten per-cent of MS patients present with a PP course of disease, which is characterized by insidious progression of symptoms from disease onset. Less than 5% of patients have PRMS and share the same poor prognosis as PPMS patients.

It has been suggested that distinct pathogenic mechanisms may be involved in different patient subgroups and have wide-ranging implications for disease classification and treatment. (Lassmann et al., 2001, Trends Mol. Med., 7, 115-121; Lucchinetti et al. 2001, Curr. Opin. Neurol., 14, 259-269). While the etiology of MS is not known, numerous studies have implicated activated monocytes and macrophages, in the presence of activated T cells, in the progression and/or exacerbation of MS. (Fredrickson et al., 1987, Acta. Neurol. Scand., 75 :352-355 *;* Huber et al., 1984, J. Exp. Med., 160 :310-316). At the microscopic level, monocytes, microglial cells (macrophages of the CNS), and activated T-cells are found within the demyelinaing regions of the nerve cells during MS exacerbations (Cecil, Textbook of Medicine (1979), Beeson et al. (eds.), W.B. Saunders Co., Philadelphia, PA.). Activated lymphocytes and macrophages have been implicated in the pathogenesis of acute inflammatory brain lesions and blood brain barrier (BBB) breakdowns associated with MS (Coles et al. 1999, Ann. Neurol. 46(3): 296-304).

In light of the association of disease progression with the presence of inflammatory cells, current medications for MS that are disease-modifying treatments, i.e., modifying the course of MS, work by modulating or suppressing the immune system. There are four FDA approved immunomodulating agents for RRMS: three beta interferons (Betaseron^{™}, Berlex; Avonex^{™}, Biogen; Rebif^{®}, Serono) and Glatimer aceate (Copaxone^{™}, Amgen). There is also one FDA approved immunosuppressing drug for worsening MS, Mitoxantrone (Novantrone^{™}, Amgen). However, it is unclear whether the drugs actually prevent disease progression or if their benefits can be sustained over time. Also, some patients can not tolerate the side effects, such as flu-like symptoms and, in some cases, hair loss, blood cell abnormalities, and depression (Fillippini et al. Interferons in relapsing remitting multiple sclerosis: a systematic review. Lancet, 361:545-552, 2003).

### a. 2-Chloro-2'-Deoxyadenosine

Various additional agents are in clinical trials for the treatment of MS. Among them, a chlorinated purine analogue 2-chloro-2'-deoxyadenosine (2-CdA) has been suggested to be useful in the treatment of MS (EP 626853B1 *and* US 5,506,214). Its mode of action may be due to its pharmacologic activity toward activated monocytes and macrophages. Several clinical studies have been described wherein 2-CdA is administered intravenously and subcutaneously to MS patients. Effective doses of an oral formulation to treat MS have also been described (US 5,506,214).

Two double-blind, placebo controlled Phase II studies using 2-CdA were conducted respectively in the treatment of progressive forms of MS (Selby et al., 1998, Can. J. Neurol. Sci., 25: 295-299) and RRS (Romine e al., 1999, Proc. Assoc. Amer. Phys., 111(1): 35-44). In the first trial, the dose was 0.1 mg/kg for 7 days by continuous intravenous injection for four months. In the second trial, the 2-CdA dose was 0.07 mg/kg for 5 days by subcutaneous injection. A placebo controlled Phase III study was conducted in patients with PPMS and SPMS. In this study, patients received 2-CdA by subcutaneous injection at a dose of 0.07 mg/kg for either two months or for six months.

The above clinical studies demonstrated the efficacy of 2-CdA treatment in MS patients in terms of Kuzke Extended Disability Status Scale (EDSS), Scripps Neurologic Rating Scale (SNRS) scores and MRI fmdings (Beutler et al., 1996, PNAS USA, 93:1716-1720*; Romine et al., 1999, supra).* The Phase II study showed 2-CdA significantly reduced MRI-measured brain lesions (*Rice et al., 2000, supra*). Some adverse effects such as increased incidence of infections related to compromised immune function or myelosuppression were observed with the highest doses (*Selby et al., 1998, supra;* Beuler et al. 1994, Acta hematol., 91: 10-15). There was a narrow margin of safety between the effective dose and the occurrence of adverse effects, so therefore the oral route of 2-CdA treatment for MS was excluded (Beutler et al., 1996, Sem. In Hematol., 33 1(S1): 45-52).

### b. Anti-Alpha-4 Integrin Antibody

An additional agent in clinical trials for the treatment of MS is a humanized monoclonal antibody specific for the alpha-4 chain of the integrin superfamily. Alpha-4-beta-1 integrin (VLA-4) is expressed on the extracellular surface of activated lymphocytes and monocytes, which have been implicated in the pathogenesis of the acute inflammatory brain lesions and blood brain barrier (BBB) breakdown associated with MS (*Coles et al. supra*). Agents against alpha-4 integrin have been tested for their anti-inflammatory potential both *in vitro* and *in vivo* in animal models (Yednock et al., 1992, Nature, 356: 63-66*;* US 5,840,229*;* US 6,001,809). The *in vitro* experiments demonstrated that anti-alpha-4 integrin antibodies block attachment of lymphocytes to brain endothelium (Tubridy et al., 1999, Neurology, 53(3), 466-472). Experiments in an animal model of MS, experimental autoimmune encephalomyelitis (EAE), demonstrated that administration of anti-alpha-4 integrin antibodies prevents inflammation of the brain and subsequent paralysis in the animals. Collectively, these experiments identify anti-alpha-4 agents as potentially useful therapeutic agents for treating MS as they prevent the trafficking of inflammatory cells across the BBB.

A Phase III trial showed that a monthly single dose of 300mg of anti-alpha-4 antibody significantly reduced MS lesions. Adverse effects seen in this study included a hypersensitivity-type reaction that was immunoglobulin-related and likely due to the fact that the agent is an antibody. Although not reported, a significant issue with protein therapeutics such as an antibody treatment is the eliciting of an immune response and subsequent development of neutralizing antibodies to the treatment making chronic administration difficult.
Current treatments for the management of MS, including the anti-alpha4 integrin antibody Antegren^{™}, are described in Leary et al (2000) Int. J. Clin. Prac, 54 (3) 161-169.

Although there have been some recent advances in treatments for MS, further improvements need to be made to facilitate the development of therapeutic agents with improved effectiveness and to reduce side effects. Accordingly, there is a need in the art for improved treatments for MS. This invention fulfills these needs and further provides other related advantages.

### SUMMARY OF THE INVENTION

This invention is directed toward the use of a novel combination of agents to treat multiple sclerosis. An aspect of the invention is set out in claim 1.

A first agent and a second agent, wherein said first agent has the pharmacological property of reducing the number of lymphocytes, and wherein said second agent blocks the adhesion of monocytes and leukocytes to endothelial cells may be administered to a patient in need thereof, wherein said first agent is 2-CdA and said second agent is an anti-alpha-4 integrin antibody.

The effective dose of either or both agents needed to provide maximal therapeutic benefit in treating multiple sclerosis may be reduced.

Pharmaceutical compositions used to treat multiple sclerosis may comprise a first agent and a second agent, wherein said first agent is 2-CdA, and wherein said second agent is an anti-alpha-4-integrin antibody which blocks the adhesion of monocytes and leukocytes to endothelial cells.

### DETAILED DESCRIPTION

This invention is related to a method of treating MS comprising administering to a patient in need thereof 2-CdA and an anti-alpha-4 integrin antibody. Although 2-CdA and anli-alpha-4 integrin antibodies have each been used individually for the treatment of MS, the agents have not been used in combination for the treatment of MS. Combining 2-CdA and an anti-alpha-4 integrin antibody is more effective in the treatment of MS than either agent administered alone. Furthermore, the combination treatment allows for a reduction in the dose of one or both of the agents required to produce a therapeutically beneficial effect or a reduction in the length of treatment required to produce a therapeutically beneficial effect. Reducing the dose of one or both of the agents may reduce adverse effects associated with administration of the individual agents, such as an immunoglobulin-related hypersensitivity reaction and/or an immune response to the anti-alpha-4 integrin antibody.

Without being bound by theory, the combination of agents may provide a synergistic effect. A synergistic effect of the agents may be due to different mechanisms of action for each of the individual agents. 2-CdA has the pharmaceutical effect of reducing lymphocyte numbers, whereas an anti-alpha-4 integrin antibody blocks the adhesion of monocytes and leukocytes to endothelial cells.
A method for treating multiple sclerosis may comprise administering to a patient in need thereof a first agent and a second agent, wherein said first agent is 2-chloro-2'-deoxyadenosine or pharmaceutically acceptable salts thereof, and wherein said second agent is an anti-alpha-4 integrin antibody which blocks the adhesion of monocytes and leukocytes to endothelial cells.
A method treating multiple sclerosis may comprise administering to a patient in need thereof a first agent and a second agent, wherein said first agent is 2-chloro-2' deoxyadenosine or pharmaceutically acceptable salts thereof, and wherein said second agent is an anti-alpha-4 integrin antibody that blocks the adhesion of monocytes and leukocytes to endothelial cells.
A kit may comprise the agents of claim 1 or 2 and instructions for using said agents to treat multiple sclerosis.
A composition comprising a first agent which is 2-CdA or a pharmaceutically acceptable salt thereof, and a second agent which is an anti-alpha-4 integrin antibody which blocks the adhesion of monocytes and leukocytes to endothelial cells may be used to treat multiple sclerosis.

According to another aspect, is provided a combination of active agents for the treatment of multiple sclerosis, said combination of active agents comprising:
(a) 2-chloro-2'-deoxyadenosine or pharmaceutically acceptable salts thereof.
(b) An anti-alpha-4 integrin antibody which blocks the adhesion of monocytes and leukocytes to endothelial cells.
The anti-alpha-4 integrin antibody may be a monoclonal antibody.
The anti-alpha-4 integrin antibody may be a chimeric antibody.
The anti-alpha-4 integrin antibody may be a human antibody.
The anti-alpha-4 integrin antibody may be a humanized antibody.
The anti-alpha-4 integrin antibody may be a single chain antibody.
The anti-alpha-4 integrin antibody may be a an antibody Fab fragment.
The said combination may comprise a sub-optimal dose of agent (a) and an effective dose of agent (b).
The combination may comprise an effective dose of agent (a) and a sub-optimal dose of agent (b).
The combination may comprise a sub-optimal dose of agent (a) and agent (b).
Agent (a) and agent (b) may be used simultaneously.
Agent (a) and agent (b) may be used sequentially.

According to another aspect, is provided a kit comprising (a) 2-chloro-2'-deoxyadenosine or pharmaceutically acceptable salts thereof, (b) an anti-alpha-4 integrin antibody and instructions for using said agents to treat multiple sclerosis.

According to another aspect, is provided a pharmaceutical composition comprising (a) 2-chloro-2'-deoxyadenosine or pharmaceutically acceptable salts thereof, (b) an anti-alpha-4 integrin antibody; and a pharmaceutically acceptable carrier, diluent or excipient thereof

### 1. Definitions

As used herein, "duration of treatment" refers to the period of administration of 2-CdA and an anti-alpha-4 integrin antibody according to the invention from the first day of treatment to the end of the treatment.

As used herein, "effective amount" when used in the context of a dosing regimen of 2-CdA in combination with an anti-alpha-4 integrin antibody, refers to that amount of either 2-CdA or an anti-alpha-4 integrin antibody, which if given in the absence of the other would result in the maximal treatment of MS that each respective agent could effect if given as a single agent.

As used herein, "efficacy" when used in the context of a dosing regimen refers to the effectiveness of a particular treatment regimen. Efficacy can be measured based on changes in the course of disease in response to a dosing regimen of this invention. For example, treatment of MS efficacy can be measured by the frequency of relapses in RRMS and the presence or absence of new lesions in the CNS as detected using methods such as MRI.

As used herein, "elicits an immune response" and "eliciting an immune response" refer to the production of an immunological response to an anti-alpha-4 integrin antibody in a subject upon introduction of the agent. An immune response in the subject can be characterized by serum reactivity with an anti-alpha-4 integrin antibody that is at least twice that of an untreated subject., more preferably three times that of the reactivity of an untreated subject, and even more preferably at least four times the reactivity of an untreated subject.

As used herein, "end of treatment" refers to the day on which the patient becomes free of 2-CdA and/or an anti-alpha-4 integrin antibody.

As used herein, "in combination with" where used to describe administration of 2-CdA and an anti-alpha-4 integrin antibody means that the 2-CdA may be administered prior to, together with, or after the anti-alpha-4 integrin antibody.

As used herein, "pathological inflammation" refers to an inappropriate and chronic inflammation associated with MS (especially to inhibit further demyelination). Such inflammation is characterized by a heightened response of inflammatory cells, including infiltrating leukocytes. Over time, such pathological inflammation often results in damage to tissue in the region of inappropriate inflammation, namely the tissues of the CNS.

As used herein, "remission" refers to the period during the course of MS progression wherein a patient experiences a reduction or stabilization of symptoms of the disease. This may be a period of partial healing of CNS lesions, and/or a reduction in CNS inflammation.

As used herein, "specifically binds" or "binds specifically" refer to the situation in which one member of a specific binding pair will not show any significant binding to molecules other than its specific binding partner (e.g., an affinity of about 1000X or more for its binding partner).

As used herein, "suboptimal dose" when used in the context of a dosing regimen of 2-CdA in combination with an anti-alpha-4 integrin antibody, refers to doses of either 2-CdA or an anti-alpha-4 integrin antibody that is less than the effective amount as defined supra

As used herein, "substantially homologous" refers to any polypeptide that has an alteration in the sequence such that a functionally equivalent amino acid is substituted for one or more amino acids in the polypeptide, thus producing a change that has no or relatively little effect on the binding properties of the polypeptide. For example, one or more amino acid residues within the sequence can be substituted by another amino acid of a similar polarity.

As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it; (b) inhibiting the disease, i.e., arresting its development; or relieving the disease, i.e., causing regression of the disease and/or its symptoms or conditions.

### 2. Combination

According to this invention 2-CdA is administered in combination with an anti-alpha-4 integrin antibody. The dosage of the agents may each be an effective amount. Preferably, the dosage of at least one of the agents is a suboptimal amount. More preferably, the dosage of each of the agents is as suboptimal amount.

The two agents may be administered simultaneously or sequentially with respect to each other. When administered simultaneously, the agents may be formulated in the same composition or in different compositions. When the two agents are not administered as part of the same formulation, the two agents may be administered by the same or different routes of administration. The relative administration of 2-CdA and an anti-alpha-4 integrin antibody may be dependent on the specific route of administration and/or formulation of each component. The relative administration of 2-CdA and an anti-alpha-4 integrin antibody may be different depending on whether the patient is experiencing remission or exacerbation of symptoms.

The combination of agents may be administered for one or more treatment cycles. Each treatment cycle may be of any duration including, but not limited to, from about two months to six months, wherein 2-CdA is given about 1-2 times per day for a total of about 5-7 days per month and an-anti-alpha-4 integrin antibody is administered about once per monthly cycle, or about 1-4 times per monthly cycle or about once per week. The number of monthly cycles may also be from about 6 to about 12, or may be more than 12. The number of monthly cycles may vary depending on the course of MS (e.g. whether the patient is experiencing RR- or SPMS) and whether the patient is experiencing remission or exacerbation of symptoms.

### 3. 2-CdA

2-CdA and its pharmacologically acceptable salts may be used in the practice of this invention. Processes for preparing 2-CdA are well known in the art. For example, the preparation of 2-CdA is described in *European Patent Application No.* 173,059 A2 and Robins et al., J. Am. Chem. Soc., 1984, 106: 6379*,* WO 04/028462*,* US 5,208,327 *and* WO 00/64918*.* Alternatively, pharmaceutical preparations of 2-CdA may be purchased from Bedford Laboratories, Bedford, Ohio.

In general, the dosage of 2-CdA may vary over a relatively wide range to achieve, and preferably maintain the desired plasma concentration. The dosage of 2-CdA may be sufficient to provide a concentration in the patient's plasma of about 0.5 nM to about 50 nM, preferably about 1 nM to about 10 nM. An elective daily dose of 2-CdA may be about 0.04 to about 1.0 mg/kg body weight, more preferably about 0.04 to about 0.20 mg/kg/day, and most preferably about 0.1mg/kg body weight. Additional dosage of 2-CdA is described in US 5,506,214.

### 4. Anti-Alpha-4 Integrin Antibodies

The anti-alpha-4 integrin antibody may specifically bind to an integrin comprising an alpha-4 subunit and may inhibit activity of the integrin. The antibody may also bind to an integrin dimer comprising the alpha-4 integrin, e.g., alpha-4-beta-1 or alpha-4-beta-7. The anti-alpha-4 integrin antibody may not show significant binding to any polypeptide other than an alpha-4 integrin or a receptor comprising an alpha-4 integrin. The antibodies may bind to an alpha-4 integrin with a binding affinity of 10⁷ mole/l or more, preferably 10⁸ mole/liters or more.

In general, the dosage of an anti-alpha-4 integrin antibody may vary over a relatively wide range to achieve, and preferably maintain the desired plasma concentration. An effective dose of an anti-alpha-4 integrin antibody is that which provides maximal reduction and/or suppression of MRI-detected lesions in the brain. The dosage of the antibody may be less than about 300 mg administered per month for a period of at least 6 months, more preferably 12 months and perhaps over the course of several years. Another preferred dosing regimen is 3 mg/kg of patient weight per month. Additional dosages of anti-alpha-4 integrin antibodies are described in U.S. Patent Publication 2004/0009169.

The antibodies of this invention include antibodies of classes IgG, IgM, IgA, IgD, and IgE, and fragments and derivatives thereof including Fab and F(ab')₂. The antibodies may also be recombinant antibody products including, but not limited to, single chain antibodies, chimeric antibody products, human antibodies, "humanized" antibody products, and "primatized" antibody products, CDR-grafted antibody products. The antibodies of this invention include monoclonal antibodies, polyclonal antibodies, affinity purified antibodies, or mixtures thereof which exhibit sufficient binding specificity to anti-alpha-4 integrin. The antibody may also be an antibody fragment. The antibody may be produced using standard techniques, including as described in WO 01/55210.

The antibody many also be attached to a label. Labels can be signal-generating enzymes, antigens, other antibodies, lectins, carbohydrates, biotin, avidin, radioisotopes, toxins, heavy metals, and other compositions known in the art. Attachment techniques are also well known in the art.

Additional antibodies may be identified using techniques available in the art. For example, monoclonal antibodies of this invention can be produced using phage display technology. Antibody fragments that selectively bind to an alpha-4 integrin or a dimer comprising an alpha-4 integrin are then isolated. Exemplary preferred methods for producing such antibodies via phage display arc disclosed in U.S. Pat. Nos. 6,225,447*;* 6,180,336*;* 6,172,197*;* 6,140,471*;* 5, 969,108*;* 5, 885,793*;* 5,872,215*;* 5,871,907*;* 5,858,657*;* 5,837,242*;* 5,733, 743*; and* 5,565, 332.

The antibodies may be provided by administering a polynucleotide encoding a whole or partial antibody (c. g., a single chain Fv) to a patient. The polynucleotide is administered to a subject in an appropriate vehicle to allow the expression of the antibody in the subject in a therapeutically effective amount.

### 5. Compositions

Compositions of this invention may further comprise one or more pharmaceutically acceptable additional ingredient(s) such as alum, stabilizers, antimicrobial agents, buffers, coloring agents, flavoring agents, adjuvants, and the like.

Compositions of this invention may be in the form of tablets or lozenges formulated in a conventional manner. For example, tablets and capsules for oral administration may contain conventional excipients including, but not limited to, binding agents, fillers, lubricants, disintegrants and wetting agents. Binding agents include, but are not limited to, syrup, accacia, gelatin, sorbitol, tragacanth, mucilage of starch and polyvinylpyrrolidone. Fillers include, but are not limited to, lactose, sugar, microcrystalline cellulose, maizestarch, calcium phosphate, and sorbitol. Lubricants include, but are not limited to, magnesium stearate, stearic acid, talc, polyethylene glycol, and silica. Disintegrants include, but are not limited to, potato starch and sodium starch glycollate. Wetting agents include, but are not limited to, sodium lauryl sulfate). Tablets may be coated according to methods well known in the art.

Compositions of this invention may also be liquid formulations including, but not limited to, aqueous or oily suspensions, solutions, emulsions, syrups, and elixirs. The compositions may also be formulated as a dry product for constitution with water or other suitable vehicle before use. Such liquid preparations may contain additives including, but not limited to, suspending agents, emulsifying agents, nonaqueous vehicles and preservatives. Suspending agent include, but are not limited to, sorbitol syrup, methyl cellulose, glucose/sugar syrup, gelatin, hydroxyethylcellulose, carboxymethyl cellulose, aluminum stearate gel, and hydrogenated edible fats. Emulsifying agents include, but are not limited to, lecithin, sorbitan monooleate, and acacia. Nonaqueous vehicles include, but are not limited to, edible oils, almond oil, fractionated coconut oil, oily esters, propylene glycol, and ethyl alcohol. Preservatives include, but are not limited to, methyl or propyl p-hydroxybenzoate and sorbic acid.

Compositions of this invention may also be formulated as suppositories, which may contain suppository bases including, but not limited to, cocoa butter or glycerides. Compositions of this invention may also be formulated for inhalation, which may be in a form including, but not limited to, a solution, suspension, or emulsion that may be administered as a dry powder or in the form of an aerosol using a propellant, such as dichlorodifluoromethane or trichlorofluoromethane. Compositions of this invention may also be formulated transdermal formulations comprising aqueous or nonaqueous vehicles including, but not limited to, creams, ointments, lotions, pastes, medicated plaster, patch, or membrane.

Compositions of this invention may also be formulated for parenteral administration including, but not limited to, by injection or continuous infusion. Formulations for injection may be in the form of suspensions, solutions, or emulsions in oily or aqueous vehicles, and may contain formulation agents including, but not limited to, suspending, stabilizing, and dispersing agents. The composition may also be provided in a powder form for reconstitution with a suitable vehicle including, but not limited to, sterile, pyrogen-free water.

Compositions of this invention may also be formulated as a depot preparation, which may be administered by implantation or by intramuscular injection. The compositions may be formulated with suitable polymeric or hydrophobic materials (as an emulsion in an acceptable oil, for example), ion exchange resins, or as sparingly soluble derivatives (as a sparingly soluble salt, for example).

Compositions of this invention may also be formulated as a liposome preparation. The liposome preparation can comprise liposomes which penetrate the cells of interest or the stratum corneum, and fuse with the cell membrane, resulting in delivery of the contents of the liposome into the cell. For example, liposomes such as those described in U.S. Patent No. 5, 077,211 of Yarosh*,* U.S. Patent No. 4,621,023 of Redziniak et al. *or* U.S. Patent No. 4,508,703 of Redziniak et al. can be used. Other suitable formulations can employ niosomes. Niosomes are lipid vesicles similar to liposomes, with membranes consisting largely of non-ionic lipids, some forms of which are effective for transporting compounds across the stratum corneum.

Representative formulations of 2-CdA are described in US 6,194,395 *and* US 5,506,214*,* WO 96/19230*,* WO 96/19229*,* WO 04/087100 *and* WO 04/087101.

### 6. Administration

Compositions of this invention may be administered in any manner including, but not limited to, orally, parenterally, sublingually, transdermally, rectally, transmucosally, topically, via inhalation, via buccal administration, or combinations thereof. Parenteral administration includes, but is not limited to, intravenous, intraarterial, intraperitoneal, subcutaneous, intramuscular, intrathecal, and intraarticular. The compositions of this invention may also be administered in the form of an implant, which allows slow release of the compositions as well as a slow controlled i.v. infusion.

This invention is further illustrated by the following examples that are not intended to limit the scope of the invention in any way.

### Example 1

### Treatment of Multiple Sclerosis

A study of sixty patients with chronic multiple sclerosis is undertaken. Each patient is first examined for normal hepatic, renal, and bone marrow functioning to establish baseline values. Patients are randomly assigned to one of the treatment groups listed in Table 1.

**Table 1**

| Group | 2-CdA | Natalizumab |
|---|---|---|
| 1 | - | - |
| 2 | 0.25 mg/kg | - |
| 3 | 0.5 mg/kg | - |
| 4 | 1 mg/kg | - |
| 5 | - | 1 mg/kg |
| 6 | - | 3 mg/kg |
| 7 | - | 6 mg/kg |
| 8 | 0.25 mg/kg | 1 mg/kg |
| 9 | 0.5 mg/kg | 3 mg/kg |
| 10 | 1 mg/kg | 6 mg/kg |

Each of the patients in Groups 2-4 and 8-10 is treated with 2-CdA dissolved in sterile preservative-free isotonic saline, whereas patients in Groups 1 and 5-7 receive a placebo (saline). The 2-CdA is administered intravenously for a total of seven days, once per month for a total of one year. Each of the patients in Groups 5-10 is treated with natalizumab, whereas Groups 1-4 received placebo (saline). The natalizumab is administered intravenously for a total of seven days, once per month for a total of one year, with the treatment being on the same day as 2-CdA treatment.
Patients are monitored to determine whether there is any progression or improvement of brain lesions associated with progression of MS. All patients have a baseline and MRI study (brain or spinal cord, according to localization of the lesions) at month 12.
Patients in Groups 1-3 and 5 have an increase in brain lesions. Patients in Groups 4 and 6-10 have a decrease in brain lesions. Importantly, patients in Group 10 show the largest decrease in brain lesions. Moreover, patients in Group 8 have a decrease in brain lesions similar to that of those patients in Groups 4 and 6. This data shows that the combination of 2-CdA and natalizumab is more effective than either agent administered alone. In addition, the data show that the combination of 2-CdA and natalizumab is effective at dosages that are ineffective for either 2-CdA or natalizumab administered alone.

## Claims

1. A combination of active agents for the treatment of multiple sclerosis, said combination of active agents comprising:
(a) 2-chloro-2'-deoxyadenosine or pharmaceutically acceptable salts thereof; and,
(b) an anti-alpha-4 integrin antibody which blocks the adhesion of monocytes and leukocytes to endothelial cells.

2. A combination according to claim 1 wherein the anti-alpha-4 integrin antibody is a monoclonal antibody.

3. A combination according to claim 1 wherein the anti-alpha-4 integrin antibody is a chimeric antibody.

4. A combination according to claim 1 wherein the anti-alpha-4 integrin antibody is a human antibody.

5. A combination according to claim 1 wherein the anti-alpha-4 integrin antibody is a humanized antibody.

6. A combination according to claim 1 wherein the anti-alpha-4 integrin antibody is a single chain antibody.

7. A combination according to claim 1 wherein the anti-alpha-4 integrin antibody is an antibody Fab fragment.

8. A combination according to any of claims 1 to 7 wherein said combination comprises 0.04 to 1 mg/kg of agent (a).

9. A combination according to any of claims 1 to 8 wherein said combination comprises 3mg/kg of agent (b).

10. A combination according to any of claims 1 to 7 wherein said combination comprises a 0.25 mg/kg of agent (a) and 3mg/kg of agent (b).

11. A combination according to any of claims 1 to 7 wherein said combination comprises 1mg/kg of agent (a) and a 1mg/kg of agent (b).

12. A combination according to any of claims 1 to 7 wherein said combination comprises a 0.25mg/kg of agent (a) and 1 mg/kg of agent (b).

13. A combination according to any of the preceding claims wherein agent (a) and agent (b) are used simultaneously.

14. A combination according to any of claims 1 to 12 wherein agent (a) and agent (b) are used sequentially.

15. A kit comprising (a) 2-chloro-2'-deoxyadenosine or pharmaceutically acceptable salts thereof, (b) an anti-alpha-4 integrin antibody which blocks the adhesion of monocytes and leukocytes to endothelial cells and instructions for using said agents to treat multiple sclerosis.

16. A pharmaceutical composition comprising (a) 2-chloro-2'-deoxyadenosine or pharmaceutically acceptable salts thereof, (b) an anti-alpha-4 integrin antibody which blocks the adhesion of monocytes and leukocytes to endothelial cells; and a pharmaceutically acceptable carrier, diluent or excipient thereof.

## Patentansprüche

1. Kombination von Wirkstoffen für die Behandlung von Multipler Sklerose, wobei die Kombination von Wirkstoffen umfasst:
(a) 2-Chlor-2'-desoxyadenosin oder pharmazeutisch verträgliche Salze davon; und
(b) einen anti-alpha-4-Integrin-Antikörper, welcher die Adhäsion von Monozyten und Leukozyten an Endothelzellen blockiert.

2. Kombination nach Anspruch 1, wobei der anti-alpha-4-Integrin-Antikörper ein monoklonaler Antikörper ist.

3. Kombination nach Anspruch 1, wobei der anti-alpha-4-Integrin-Antikörper ein chimärer Antikörper ist.

4. Kombination nach Anspruch 1, wobei der anti-alpha-4-Integrin-Antikörper ein humaner Antikörper ist.

5. Kombination nach Anspruch 1, wobei der anti-alpha-4-Integrin-Antikörper ein humanisierter Antikörper ist.

6. Kombination nach Anspruch 1, wobei der anti-alpha-4-Integrin-Antikörper ein Einzelkettenantikörper ist.

7. Kombination nach Anspruch 1, wobei der anti-alpha-4-Integrin-Antikörper ein Antikörper-Fab-Fragment ist.

8. Kombination nach einem der Ansprüche 1 bis 7, wobei die Kombination 0,04 bis 1 mg/kg von Wirkstoff (a) umfasst.

9. Kombination nach einem der Ansprüche 1 bis 8, wobei die Kombination 3 mg/kg von Wirkstoff (b) umfasst.

10. Kombination nach einem der Ansprüche 1 bis 7, wobei die Kombination 0,25 mg/kg von Wirkstoff (a) und 3 mg/kg von Wirkstoff (b) umfasst.

11. Kombination nach einem der Ansprüche 1 bis 7, wobei die Kombination 1 mg/kg von Wirkstoff (a) und 1 mg/kg von Wirkstoff (b) umfasst.

12. Kombination nach einem der Ansprüche 1 bis 7, wobei die Kombination 0,25 mg/kg von Wirkstoff (a) und 1 mg/kg von Wirkstoff (b) umfasst.

13. Kombination nach einem der vorangehenden Ansprüche, wobei Wirkstoff (a) und Wirkstoff (b) gleichzeitig verwendet werden.

14. Kombination nach einem der Ansprüche 1 bis 12, wobei Wirkstoff (a) und Wirkstoff (b) nacheinander verwendet werden.

15. Kit, umfassend (a) 2-Chlor-2'-desoxyadenosin oder pharmazeutisch verträgliche Salze davon, (b) einen anti-alpha-4-Integrin-Antikörper, welcher die Adhäsion von Monozyten und Leukozyten an Endothelzellen blockiert, und Anleitungen zur Verwendung dieser Wirkstoffe zum Behandeln von Multipler Sklerose.

16. Pharmazeutische Zusammensetzung, umfassend (a) 2-Chlor-2'-desoxyadenosin oder pharmazeutisch verträgliche Salze davon, (b) einen anti-alpha-4-Integrin-Antikörper, welcher die Adhäsion von Monozyten und Leukozyten an Endotheizeilen blockiert, und einen pharmazeutisch verträglichen Träger, ein pharmazeutisch verträgliches Verdünnungsmittel oder einen pharmazeutisch verträglichen Excipienten davon.

## Revendications

1. Combinaison d'agents actifs pour le traitement de la sclérose en plaques, ladite combinaison d'agents actifs comprenant :
(a) une 2-chloro-2'-désoxyadénosine ou des sels de celle-ci pharmaceutiquement acceptables ; et
(b) un anticorps anti-alpha-4-intégrine qui bloque l'adhérence des monocytes et des leucocytes aux cellules endothéliales.

2. Combinaison selon la revendication 1, dans laquelle l'anticorps anti-alpha-4-intégrine est un anticorps monoclonal.

3. Combinaison selon la revendication 1, dans laquelle l'anticorps anti-alpha-4-intégrine est un anticorps chimérique.

4. Combinaison selon la revendication 1, dans laquelle l'anticorps anti-alpha-4-intégrine est un anticorps humain.

5. Combinaison selon la revendication 1, dans laquelle l'anticorps anti-alpha-4-intégrine est un anticorps humanisé.

6. Combinaison selon la revendication 1, dans laquelle l'anticorps anti-alpha-4-intégrine est un anticorps monocaténaire.

7. Combinaison selon la revendication 1, dans laquelle l'anticorps anti-alpha-4-intégrine est un fragment Fab d'anticorps.

8. Combinaison selon l'une quelconque des revendications 1 à 7, dans laquelle ladite combinaison comprend de 0,04 à 1 mg/kg d'agent (a).

9. Combinaison selon l'une quelconque des revendications 1 à 8, dans laquelle ladite combinaison comprend 3 mg/kg d'agent (b).

10. Combinaison selon l'une quelconque des revendications 1 à 7, dans laquelle ladite combinaison comprend 0,25 mg/kg d'agent (a) et 3 mg/kg d'agent (b).

11. Combinaison selon l'une quelconque des revendications 1 à 7, dans laquelle ladite combinaison comprend 1 mg/kg d'agent (a) et 1 mg/kg d'agent (b).

12. Combinaison selon l'une quelconque des revendications 1 à 7, dans laquelle ladite combinaison comprend 0,25 mg/kg d'agent (a) et 1 mg/kg d'agent (b).

13. Combinaison selon l'une quelconque des revendications précédentes, dans laquelle l'agent (a) et l'agent (b) sont utilisés simultanément.

14. Combinaison selon l'une quelconque des revendications 1 à 12, dans laquelle l'agent (a) et l'agent (b) sont utilisés séquentiellement.

15. Trousse comprenant (a) une 2-chloro-2'-désoxyadénosine ou des sels de celle-ci pharmaceutiquement acceptables, (b) un anticorps anti-alpha-4-intégrine qui bloque l'adhérence des monocytes et des leucocytes aux cellules endothéliales et des instructions pour utiliser lesdits agents pour traiter la sclérose en plaques.

16. Composition pharmaceutique comprenant (a) une 2-chloro-2'-désoxyadénosine ou des sels de celle-ci pharmaceutiquement acceptables, (b) un anticorps anti-alpha-4-intégrine qui bloque l'adhérence des monocytes et des leucocytes aux cellules endothéliales et un véhicule, un diluant ou un excipient pharmaceutiquement acceptable.
